Europäisches Patentamt

European Patent Office

Office européen des brevets

(11) Publication number: **0 242 359 B1**

# EUROPEAN PATENT SPECIFICATION

(45) Date of publication of patent specification: **21.07.93**

(51) Int. Cl.⁵: **A61K 39/108**, C07G 17/00, C07K 15/12, C12N 1/20, C07K 15/00, C12P 19/34, C12P 21/00, C12N 15/68

(21) Application number: **86900017.4**

(22) Date of filing: **04.12.85**

(86) International application number: **PCT/AU85/00306**

(87) International publication number: **WO 86/03410 (19.06.86 86/13)**

(54) A CLONING VEHICLE FOR CLONING A LARGE FRAGMENT OF DNA AND A VACCINE COMPRISING 987P FIMBRIAL PROTEIN.

(30) Priority: **04.12.84 AU 8403/84**

(43) Date of publication of application:
**28.10.87 Bulletin 87/44**

(45) Publication of the grant of the patent:
**21.07.93 Bulletin 93/29**

(84) Designated Contracting States:
**BE CH DE GB IT LI NL SE**

(56) References cited:
**EP-A- 0 106 542**
**EP-A- 0 180 192**
**GB-A- 2 094 314**

**GENETIC ENGINEERING, vol. 2, 1980, pp. 169-183, B. HOHN et al.: "Cloning with cosmids in E. coli and yeast"**

**MICROBIOLOGICAL REVIEWS, vol. 46, no. 2, June 1982, pp. 129-161, W. GAASTRA et al.: "Host-specific fimbrial adhesins of noninvasive enterotoxigenic Escherichia coli**

strains"

(73) Proprietor: **BIOTECHNOLOGY AUSTRALIA PTY. LTD.**
**28 Barcoo Street**
**East Roseville, NSW 2069(AU)**

(72) Inventor: **CLARK, Susan, Joy**
**41 Bellevue Street**
**Chatswood, NSW 2067(AU)**
Inventor: **CAHILL, Ann, Denise**
**13 Cameron Avenue**
**Artarmon, NSW 2064(AU)**

(74) Representative: **Bannerman, David Gardner et al**
**Withers & Rogers 4 Dyer's Buildings Holborn London, EC1N 2JT (GB)**

## Description

The present invention relates to cloning vectors, and methods for cloning the 987P fimbrial genes and also to a vaccine containing the fimbriae produced thereby.

Background Art

Neonatal diarrhoea in piglets follows the proliferation of certain strains of E. coli in the small intestine. These certain strains synthesise enterotoxins which cause diarrhoea and dehydration which in turn frequently result in death of the piglets. These enterotoxigenic strains of E. coli are characterized by a rapid proliferation in the small intestine, which has been attributed to the ability of the bacteria to attach themselves to the intestinal epithelium via fimbriae.

A number of different fimbrial serotypes of E. coli have been associated with neonatal diarrhoea, the main ones being K88ac, K88ab, K99 and 987P. Genes for K88ac, K88ab and K99 are known to be plasmid encoded and have already been cloned. However, the genes coding for 987P fimbriae had not yet been cloned and it had not yet been determined whether these genes are plasmid or chromosomally located.

It has been considered that sows can be vaccinated with one or bore of the fimbrial antigens so that they produce antibodies thereto which in turn will prevent the enterotoxigenic E. coli from attaching to the small intestine of the piglet, multiplying and causing the symptoms associated with the infection. Therefore, it would be desirable to produce the fimbrial antigens in quantities sufficient to allow wide scale vaccination of the animals. Isolation of fimbriae from wild-type E. coli is difficult because of low numbers of fimbriae on the E. coli. However, if the genes coding for the fimbriae could be cloned into a high copy plasmid vector in a suitable host, the expression of the fimbriae could be increased 10-100 fold. These fimbriae can then be isolated in high quantities and the fimbriae so produced would also be free of the wild-type enterotoxins which could produce undesirable side effects upon vaccination.

It was always considered that cloning of 987P genes would be extremely difficult, if not impossible. Firstly, the location of the genes had not been identified but also, it was thought that very large DNA fragments would be required to enable 987P expression. Large fragments of DNA are notoriously unstable in high copy plasmid vectors.

Disclosure of the Invention

The present invention provides a method of construction of a cloning vehicle capable of expressing 987P fimbriae characterized by the introduction of a partition locus and a DNA fragment which contains the genes for expression of 987P fimbriae into a high copy number plasmid such as pBR or pACYC based vectors.

Particularly preferred plasmids are pBR322, pBR329 and pACYC184.

Work with the 987P fimbriae has led the present inventors to conclude that the insertion of a partition locus into a plasmid cloning vector, such as a pBR plasmid, allows or enhances the stable cloning of large fragments of DNA into such vectors.

It is a further object to provide recombinant plasmids, pBTA201 and pBTA200.

The invention provides a cloning vector which comprises a plasmid into which has been inserted a DNA segment which contains a partition locus and a DNA segment which contains the genes coding for 987P fimbrial protein.

The present invention also provides a method for the construction of a cloning vector capable of expressing 987P fimbriae, which method comprises:

(i) Inserting a DNA fragment containing a partition locus into a suitable plasmid, such as pBR329, pBR322, or pACYC184, and

(ii) Inserting into the plasmid of step (i), a DNA fragment which contains the genes for expression of 987P fimbriae.

The partition locus most preferably used is the sop locus isolated from the mini F plasmid pML31 as described in Austin and Wierzbicki, PLASMID, 10, 73-81, 1983.

The invention provides E. coli transformed by a cloning vehicle capable of expressing 987P fimbrial protein. In particular, it is preferred that E. coli be transformed by pBTA201.

The invention further provides a transformed microorganism, transformed by a cloning vehicle produced by the method of the invention. A very suitable microorganism for use in the present invention is E. coli.

The invention also provides a vaccine comprising E. coli modified to include the cloning vector of the present invention which is capable of expressing 987P fimbrial protein.

2

Alterntively, the vaccine of the present invention may comprise E. coli transformed by a vehicle capable of expressing 987P fimbriae.

## Brief Description of the Drawings

Fig. 1 is a DNA restriction map of the vector pBTA599.
Fig. 2 is a restriction map of pBTA201, the 52 kilobase insert and vector.

## Best Mode of Carrying Out the Invention

## Construction of Cloning Vector

The cloning vector was constructed by inserting a partition locus from the mini F plasmid pML31 into pBR329.

Plasmids derived from pBR322 lack the functions required for equal partition. This means that pBR322 is not stably maintained past 50 generations. The partition locus however codes for a mechanism that ensures the accurate partitioning of plasmids at cell division. (Meacock and Cohen, CELL, 20, S29-S42, 1980 and Tucker et al CELL 38, 191-201, 1984).

A partition locus has been shown to be located on the F plasmid of E. coli (Austin and Wierzbicki, PLASMID, 10, 73-81, 1983), and more specifically to the 46-49.4 kb region of the F plasmid (Ogura and Hiraga, Proc. Natl. Acad. Sci. USA, 80, 4784-4788 1983). This particular partition locus is known as the sop locus.

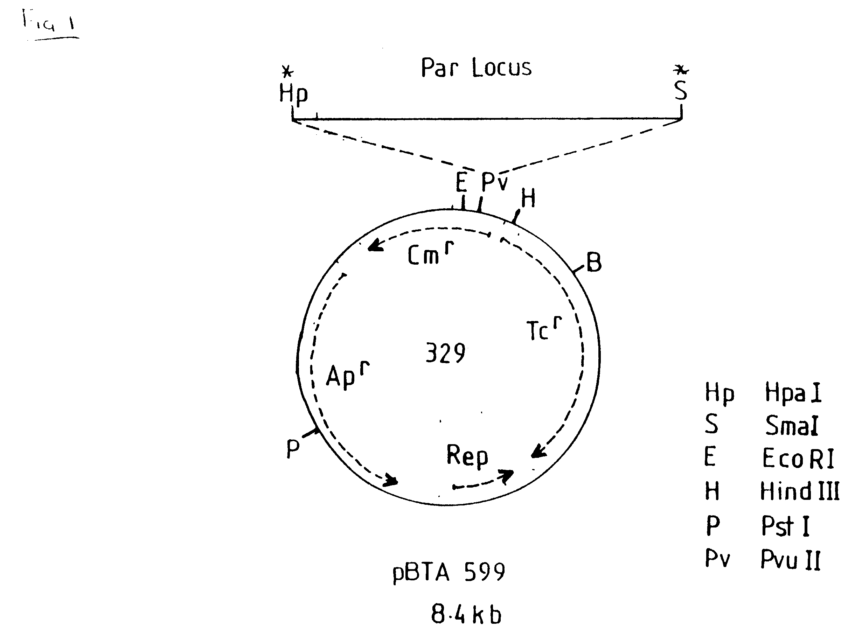

We have cloned the SmaI/HpaI fragment (45.3 kb - 49.4 kb) of the F plasmid from pML31 into the PvuII site of pBR329. Plasmid pML31 was sequentially digested with SmaI then HpaI in 5mM NaCl, 1mM Tris pH 7.4, 1mM MgSO$_4$ and 0.1 mM DTT (medium salt buffer) at 37$^o$C. The digest was loaded onto a 0.8% agarose gel and the 4.1 kb fragment eluted onto NA45 paper. A fragment of approximately 4.2 kb present in the digest coeluted with the 4.1 kb fragment. The DNA was extracted from the NA45 paper by heating at 65$^o$C for 60 minutes in TE containing 1M NaCl. The NaCl concentration was reduced to 0.2M, the DNA was phenol and chloroform extracted then finally ethanol precipitated. The pBR329 vector was PvuII digested in medium salt buffer, then phosphatased in 0.1M Tris pH8. Approximately 600ng each of eluted HpaI SmaI fragments and phosphatased pBR329 were ligated in 1mM ATP, 10mM DDT, 10mM MgCl$_2$ and 10mM Tris pH 8.0 with 1 unit of T4-DNA ligase at 4$^o$C overnight. The ligation was transformed to fresh competent CaCl$_2$ prepared MC1061 cells. Colonies were selected for the presence of pBR329 on LB + Ampicillin plates and screened for the presence of inserts on LB + Cloramphenicol plates. Plasmids from colonies containing inserts were digested with PstI and PvuI to determine that the required 4.1 kb HpaI SmaI fragment had been inserted. This fragment should contain the partition locus and therefore should not only make pBR329 stable, but also make any recombinant molecules derived from this vector stable, e.g. pBR329 containing large DNA inserts. This new vector is called pBTA599 and is as shown in Fig. 1.

## 1. ANALYSIS OF pBTA599

The stability of large fragments cloned into pBTA599 and the efficiency of transformation of these large plasmids was investigated.

In construction of the 987P plasmid DNA library a 60.4kb plasmid (pBTA201) was isolated. A 33kb BamHI fragment from pBTA201 was subcloned into pBTA599 to generate a 41.3kb plasmid (pBTA360). The transformation efficiency of these plasmids was compared with pBR322, and pBTA599. Approximately 10 ng of each plasmid was transformed into 200ul of fresh competent CaCl$_2$ prepared MC1061 cells. The results are summarised in Table 1.

TABLE 1

| Plasmid | Plasmid size | Efficiency of Transformation Colonies/ug |
|---------|--------------|------------------------------------------|
| pBR322  | 4.2kb        | $2.0 \times 10^6$                        |
| pBTA599 | 8.4kb        | $3.38 \times 10^5$                       |
| pBTA360 | 41.3kb       | $2.4 \times 10^4$                        |
| pBTA201 | 60.4kb       | $1 \times 10^3$                          |

3

As the plasmid size increased the transformation efficiency decreased. The transformation efficiency of the 60.4kb plasmid pBTA201 compared to pBR322 was only 3 logs lower, which easily allows for the cloning of large DNA fragments. Both the 52kb and 33kb DNA inserts from pBTA201 and pBTA360 were also stably maintained through transformation and multiple generations.

Location of the 987P genes

In order to determine if the genes encoding the 987P fimbriae were chromosomally or plasmid encoded, the following experiment was done.

A wild type isolate of 987P was mated with a K12 NxR strain of E coli by a quantitative broth method. Log phase cultures of the wild type donor and K12 recipient were mixed in the proportion of 1:10 with the recipient in excess. The mating mixture was incubated statically at 37°C for 1 hour then mating pairs were disrupted by vortexing. The mating mixture was plated on sheep blood agar + Naladixic Acid. screening for transconjugants was by testing for 987P expression by agglutination using 987P antiserum. A number of positive colonies were isolated. These were shown to contain part of the plasmid content of the wild-type 987P strain. This means that the 987P genes are encoded on plasmid DNA and not on chromosomal DNA.

Construction of a plasmid DNA library from wild-type 987P E.coli

Plasmid DNA was isolated from a wild-type isolate of 987P E. coli by a modified method of Hansen and Olsen (J. Bacteriol 135, 227-238, 1978) to ensure high yields of large plasmids.

Partial BamHI digests of the plasmid DNA were performed to generate DNA fragments larger than 30 kb. The digestion was performed in medium salt buffer with aliquots being removed at 15. 30, 45 and 60 minutes digestion. The 15 minute digestion yielded the required fragment sizes. The pBTA599 vector was BamHI digested in medium salt buffer then phosphatased in 0.1M Tris pH 8. The partial 987P digest was ligated to the phosphatased vector in 1mM ATP, 10mM Tris pH 8, 10mM $MgCl_2$ and 10mM DTT with 1 unit of T4-DNA ligase at 4°C overnight. Approximately 250ng vector was ligated to approximately 5ug partial digest. The ligation was transformed to fresh $CaCl_2$ prepared competent MC1061 cells. Approximately 4000 colonies were generated which were ApR and 94% of these were Tet sensitive indicating they contained an inserted DNA fragment. Approximately 500 of the colonies were screened for 987P expression by colony immunoassay using antiserum specific for 987P fimbriae.

Four positive colonies were detected and these represent two types. Two colonies contain a 68 Kb insert (pBTA200) and the other two contain a 52 Kb insert (pBTA201).

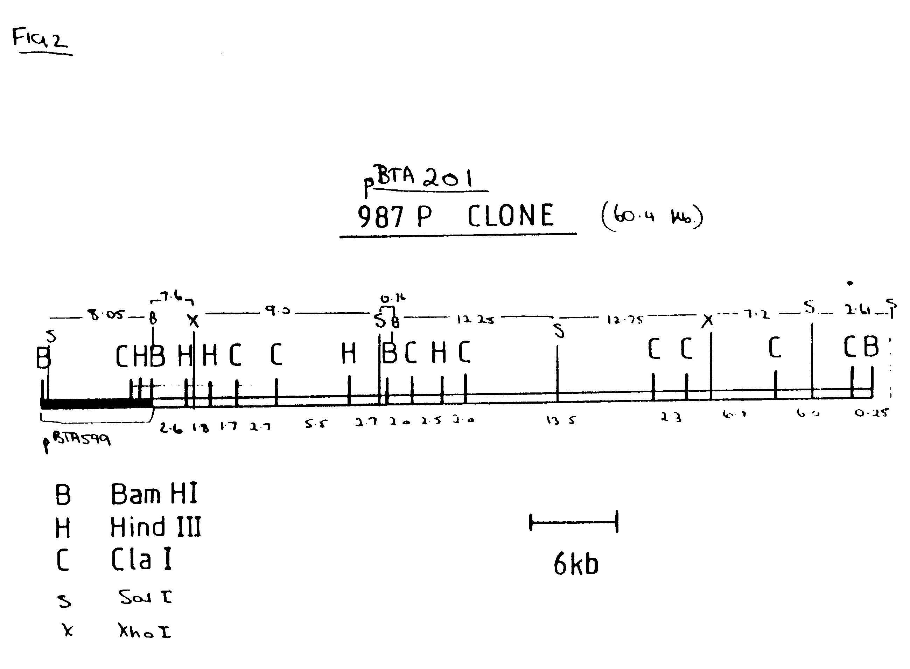

A restriction map of the 52 Kb insert and vector (pBTA201) is shown in Fig. 2.

The 987P operon may span a large area of the pBTA201 insert as no subclones from pBTA201 were isolated which still expressed 987P fimbriae.

987P VACCINE PRODUCTION

Host cells which contain recombinant plasmid expressing 987P fimbriae and assembly genes are maintained as freeze dried vials in the production culture collection. Cells from the storage vials are reconstituted and plated out for single colonies on selective nutrient agar medium. After growth, 987P expressing colonies are identified by sero-agglutination and the most active colonies are used to produce fermenter inocula. The inoculum is used to seed a fermenter which contains a medium comprising tryptone yeast extract salts and carbon source. The fermentation is monitored by standard methods and practices used by those skilled in the art. Upon completion, the fimbrial product is separated from the bacterial cell in situ and the waste biomass is removed by centrifugation. The resultant fimbrial suspension is washed with buffer solutions and concentrated to its final aqueous form using hollow fibre ultrafiltration.

The aqueous concentrate is stored with preservatives at 4°C after Quality Control and titre analyses.

Formulation of the water in oil emulsion vaccine is achieved by aseptically metering in a precise volume of 987P aqueous concentrate along with other sterile antigen components and preservatives. The water phase is made up to a volume with sterile water and is equal to that of the sterile oil phase. The completed water phase is then added at a pre-determined rate to the oil phase during high speed homogenisation to give the final formulation.

Vaccine Trials

A series of controlled challenge trials using 987P[+] Enterotoxigenic Escherichia coli (ETEC) were conducted in piglets suckling unvaccinated sows or sows vaccinated with an E.coli vaccine containing 987P fimbrial antigen. The aims of the trial were to demonstrate:

a. Protection of piglets suckling vaccinated sows from diarrhoea and death caused by 987P ETEC.

b. Reduced colonization of small intestine by 987P ETEC in piglets suckling vaccinated sows.

c. Substantial antibody titres in colostrum and serum of sows vaccinated with vaccine and in serum of their piglets.

Methodology

The vaccine used in the controlled challenge trials contained the four purified fimbrial antigens, K88ac, K88ab, K99 and 987P, with an oil adjuvant.

Sows were inoculated intramuscularly twice during pregnancy (8 weeks and 1-2 weeks before farrowing) with the vaccine or a placebo.

Piglets were allowed to suckle naturally before challenge. At between 5 and 13 hours old, they were dosed orally with a mixture of two enterotoxigenic E. coli strains expressing 987P fimbriae. The challenge inoculum contained $1 \times 10^9$ viable bacteria of each strain.

Initial diagnosis of diarrhoea was by visual assessment. Selected piglets were necropsied 16 to 28 hours after challenge depending on presence and severity or absence of scouring. The following laboratory tests were performed to confirm the cause of diarrhoea:

a. Bacterial counts from proximal, middle and distal sections of small intestine.

b. Gut histopathology from the same sites to identify pathologic changes and presence of bacteria adhering to enterocytes.

c. Level of digestive enzymes (alkaline phosphatase, lactase) as an indication of structural mucosal cell damage (e.g. in rotavirus diarrhoea, the concentrations of these enzymes are greatly reduced).

Specific anti-fimbriae IgG antibody titres were determined by Enzyme Linked Immunosorbent Assay.

Results

Severe diarrhoea occurred in 100% of piglets born to control sows while 22.5% of piglets from vaccinated sows suffered mild transient looseness only (Table 2). The observed difference between the two groups was highly significant ($x^2 = 40.9$, $p<0.005$).

Table 2

| EFFICACY OF VACCINE AGAINST 987P ETEC CHALLENGE | | | |
|---|---|---|---|
| Treatment Group | No. of Sows | No. of Piglets | No. of Piglets Scouring (%) |
| Control | 3 | 26 | 26(100)[a] |
| Vaccinated | 4 | 40 | 9(22.5)[a] |

a. Significant difference ($x^2$ - 40.9, $p<0.005$)

Scouring in all control piglets was very severe and the resultant dehydration (about 10%) was life-threatening. Consequently, remaining control piglets were destroyed 32 to 36 hours after challenge. By contrast, none of the piglets from vaccinated sows were scouring when the trial was terminated 46 to 48 hours after challenge.

Colonization of the distal small intestine, as indicated by bacterial counts of mucosal scrapings, was highly significantly reduced in piglets suckling vaccinated sows compared to piglets suckling placebo-inoculated sows ($t = 4.7$, 14 d.f., $p<0.005$).

Histopathology on gut sections from challenged piglets revealed extensive adhesion of coliform bacteria to the mucosa of the small intestine in all control piglets, while in piglets from vaccinated sows, there was little or no attachment of coliforms to the mucosa.

No virus involvement in the disease syndrome was implicated on the basis of digestive enzyme assays and histopathology.

High specific anti-987P antibody titres were demonstrated in samples of serum and colostrum from vaccinated sows as well as serum samples from their piglets whilst placebo-inoculated sows and their piglets had very low antibody levels (Table 3). The difference between the mean titre of piglets suckling vaccinated sows and the mean titre of piglets on control sows was highly significant e.g. t = 15.7, 46d.f., p<0.001.

Table 3

SEROLOGICAL RESPONSES IN SOWS AND PIGLETS

TO E. COLI VACCINE

| | | Anti-987P IgG Titre[a] | | | | |
|---|---|---|---|---|---|---|
| Sow No. | Treatment | Sow Pre-Vacc Serum | Sow Post-Farrowing | | Mean Piglet Serum[b] | 95%C.I. Piglet Titres[c] |
| | | | Serum | Colostrum | | |
| 133 | Control | nd | 70 | nd | <31 | 28- 36 |
| 164 | Control | 54 | 58 | 136 | <28 | 11- 71 |
| 187 | Control | <25 | 50 | 117 | <34 | 27- 42 |
| 110 | Vacc. | 50 | 1009 | 906 | 304 | 208- 445 |
| 182 | Vacc. | 30 | 521 | 819 | 237 | 159- 353 |
| 206 | Vacc. | 94 | 737 | 562 | 599 | 468- 767 |
| 231 | Vacc. | <25 | 2407 | 869 | 895 | 733-1094 |

a.   Reciprocal of maximum serum dilution in ELISA.

b.   Geometric mean titre.  To permit calculation of geometric means, the lowest serum dilutions tested were used as the titres for sample negative by ELISA. Means which include such samples are preceded by <.

c.   95% C.I. (confidence interval):  the range within which 95% of titres will fall $(\bar{x} \pm 1.96 \ s/\sqrt{n})$.

nd   experiment was not carried out.

It is concluded from these results that piglets obtaining adequate amounts of colostrum from vaccinated sows will be solidly protected from 987P ETEC colonization of intestinal mucosa and consequent diarrhoea.

**Claims**

1.   A method of construction of a cloning vehicle capable of expressing 987P fimbriae characterised by the introduction of a partition locus and a DNA fragment which contains the genes for expression of 987P into a high copy number plasmid.

2.   A method of constructions of a cloning vehicle capable of expressing 987P fimbriae according to claim 1, which method comprises:
(i) inserting a DNA fragment containing a partition locus into a high copy number plasmid, and
(ii) inserting into the plasmid of step (i), a DNA fragment which contains the genes for expression of 987P fimbriae.

3.   A method according to claim 1 or 2 wherein the high copy number plasmid is of the pBR type or the pACYC type.

EP 0 242 359 B1

4.  A method according to claim 3 wherein the plasmid is pBR322.

5.  A method according to claim 3 wherein the plasmid is pBR329.

6.  A method according to claim 3 wherein the plasmid is pACYC184.

7.  A method according to claim 2 wherein the partition locus is the sop locus isolated from the mini F plasmid pML31.

8.  The cloning vehicle obtainable from the method of any one of claims 1 to 7.

9.  A recombinant cloning vector comprising a DNA segment containing a partition locus and a DNA segment which contains the genes coding for 987P fimbrial protein.

10. A recombinant plasmid, of 60.4 kb in size and consisting of pBR329 having the sop locus of the mini F plasmid pML31 as a 4.1 kb HpaI/SmaI fragment inserted into the PvuII site of the chloramphenicol resistance gene and a 52 kb partial BamHI fragment of plasmid DNA carrying genes coding for expression of 987P from a wild type isolate of 987P E. coli inserted into the BamHI site of the tetracyclene resistance gene.

11. A recombinant plasmid, of 76.4 kb in size, consisting of pBR329 having the sop locus of the mini F plasmid pML31 as a 4.1 kb HpaI/SmaI fragment inserted into the PvuII site of the chloramphenicol resistance gene and a 68 kb partial BamHI fragment of plasmid DNA carrying genes coding for expression of 987P from a wild-type isolate of 987P E. coli inserted into the BamHI site of the tetracycline resistance gene.

12. A microorganism obtainable by transformation with a cloning vehicle according to claim 8.

13. A microorganism according to claim 12 wherein said microorganism is E. coli.

14. A microorganism according to claims 12 or 13 wherein the cloning vehicle is A recombinant plasmid according to claim 10.

15. A vaccine comprising E. coli transformed by a cloning vehicle produced by the method defined by any one of claims 1 to 7.

16. A vaccine comprising E. coli transformed by a cloning vector according to claim 9 capable of expressing 987P fimbrial protein.

17. A method of producing a vaccine comprising 987P fimbriae characterised in that the method includes the step of transforming E. coli with a cloning vector according to claim 9.

**Patentansprüche**

1.  Verfahren zur Herstellung eines Klonierungsvehikels, das zur Expression von 987P-Fimbrien befähigt ist, gekennzeichnet durch das Einfügen eines Partitions-Locus und eines DNA-Fragments, welches die Gene für die Expression von 987P enthält, in ein Plasmid mit hoher Kopienzahl.

2.  Verfahren zur Herstellung eines Klonierungsvehikels, das zur Expression von 987P-Fimbrien befähigt ist, nach Anspruch 1, gekennzeichnet durch:
    (i) Einfügen eines DNA-Fragments, das einen Partitions-Locus enthält, in ein Plasmid mit hoher Kopienzahl, und
    (ii) Einfügen eines DNA-Fragments, das die Gene für die Expression von 987P-Fimbrien enthält, in das Plasmid des Schrittes (i).

3.  Verfahren gemäß Anspruch 1 oder 2, dadurch gekennzeichnet, daß das Plasmid mit hoher Kopienzahl vom pBR-Typ oder pACYC-Typ ist.

**4.** Verfahren gemäß Anspruch 3, dadurch gekennzeichnet, daß das Plasmid pBR322 ist.

**5.** Verfahren gemäß Anspruch 3, dadurch gekennzeichnet, daß das Plasmid pBR329 ist.

**6.** Verfahren gemäß Anspruch 3, dadurch gekennzeichnet, daß das Plasmid pACYC184 ist.

**7.** Verfahren gemäß Anspruch 2, dadurch gekennzeichnet, daß der Partition-Locus der sop-Locus, isoliert aus dem Mini-F-Plasmid pML31, ist.

**8.** Klonierungsvehikel, erhältlich nach einem Verfahren gemäß eines jeden der Ansprüche 1 bis 7.

**9.** Rekombinanter Klonierungsvektor, enthaltend ein DNA-Segment, das einen Partitions-Locus und ein DNA-Segment aufweist, welches die Gene einschließt, die für 987P-Fimbrienprotein kodieren.

**10.** Rekombinantes Plasmid mit einer Größe von 60,4 kb und bestehend aus pBR329 mit dem sop-Locus des Mini-F-Plasmids pML31 als ein 4,1 kb-HpaI/SmaI-Fragment, insertiert in den PvuII-Ort des Chloramphenicol-Resistenzgens, sowie einem für die Expression von 987P eines Wildtyp-Isolats von 987P-E.-coli kodierende Gene tragendem 52 kb-Partial-BamHI-Fragment einer Plasmid-DNA, insertiert in den BamHI-Ort des Tetracyclin-Resistenzgens.

**11.** Rekombinantes Plasmid mit einer Größe von 76,4 kb und bestehend aus pBR329 mit dem sop-Locus des Mini-F-Plasmids pML31 als ein 4,1 kb-HpaI/SmaI-Fragment, insertiert in den PvuII-Ort des Chloramphenicol-Resistenzgens, sowie einem für die Expression von 987P eines Wildtyp-Isolats von 987P-E.-coli kodierende Gene tragendem 68 kb-Partial-BamHI-Fragment einer Plasmid-DNA, insertiert in den BamHI-Ort des Tetracyclin-Resistenzgens.

**12.** Mikroorganismus, erhältlich durch die Transformation mit einem Klonierungsvehikel gemäß Anspruch 8.

**13.** Mikroorganismus gemäß Anspruch 12, wobei der genannte Mikroorganismus E. coli ist.

**14.** Mikroorganismus gemäß Anspruch 12 oder 13, wobei das Klonierungsvehikel ein Plasmid gemäß Anspruch 10 ist.

**15.** E. coli enthaltender Impfstoff, transformiert durch ein Klonierungsvehikel, hergestellt nach einem Verfahren eines jeden der Ansprüche 1 bis 7.

**16.** E. coli enthaltender Impfstoff, transformiert durch einen Klonierungsvektor gemäß Anspruch 9, der für die Expression von 987P-Fimbrien Protein geeignet ist.

**17.** Verfahren zur Herstellung eines Impfstoffes, der 987P-Fimbrien enthält, dadurch gekennzeichnet, daß das Verfahren den Schritt der Transformierung von E. coli mit einem Klonierungsvektor gemäß Anspruch 9 einschließt.

**Revendications**

**1.** Méthode pour construire un véhicule de clonage susceptible d'exprimer des fimbrae 987P, caractérisé en ce qu'on introduit un locus de partition et un fragment d'ADN qui contient les gènes d'expression de 987P dans un plasmide à nombre de copies élevé.

**2.** Méthode pour construire un véhicule de clonage susceptible d'exprimer des fimbrae 987P selon la revendication 1, laquelle méthode comprend les étapes suivantes :
  (i) introduire un fragment d'ADN contenant un locus de partition dans un plasmide à nombre de copies élevé, et
  (ii) introduire dans le plasmide de l'étape (i) un fragment d'ADN qui contient les gènes d'expression des fimbrae 987P.

**3.** Méthode selon l'une des revendications 1 ou 2, dans laquelle le plasmide à nombre de copies élevé est du type pBR ou du type pACYC.

4. Méthode selon la revendication 3, dans laquelle le plasmide est pBR322.

5. Méthode selon la revendication 3, dans laquelle le plasmide est pBR329.

6. Méthode selon la revendication 3, dans laquelle le plasmide est pACYC184.

7. Méthode selon la revendication 2, dans laquelle le locus de partition est le locus sop isolé du miniplasmide F pML31.

8. Véhicule de clonage pouvant être obtenu par la méthode selon l'une des revendications 1 à 7.

9. Vecteur de clonage recombiné comprenant un segment d'ADN contenant un locus de partition et un segment d'ADN qui contient les gènes codant pour la protéine des fimbrae 987P.

10. Plasmide recombiné d'une dimension de 60,4 kb et comprenant un pBR329 ayant le locus sop du miniplasmide F pML31 comme fragment HpaI/SmaI de 4,1 kb introduit dans le site PvuII du gène de résistance au chloramphénicol et un fragment BamHI de 52 kb de l'ADN du plasmide portant les gènes codant pour l'expression de 987P en provenance d'un isolat de type sauvage de E. Coli 987P introduit dans le site BamHI du gène de résistance à la tétracycline.

11. Plasmide recombiné d'une dimension de 76,4 kb, comprenant un pBR329 ayant le locus sop du miniplasmide F pML31 comme fragment HpaI/SmaI de 4,1 kb introduit dans le site PvuII du gène de résistance au chloramphénicol et un fragment BamHI partiel de 68 kb de l'ADN du plasmide portant les gènes codant pour l'expression de 987P en provenance d'un isolat de type sauvage de E. Coli 987P introduit dans le site BamHI du gène de résistance à la tétracycline.

12. Microorganisme pouvant être obtenu par transformation avec un véhicule de clonage selon la revendication 8.

13. Microorganisme selon la revendication 12, qui est E. Coli.

14. Microorganisme selon l'une des revendications 12 ou 13, dans lequel le véhicule de clonage est un plasmide recombiné selon la revendication 10.

15. Vaccin contenant E. Coli transformé par un véhicule de clonage produit par la méthode selon l'une quelconque des revendications 1 à 7.

16. Vaccin contenant E. Coli transformé par un vecteur de clonage selon la revendication 9 susceptible d'exprimer la protéine de fimbrae 987P.

17. Méthode pour produire un vaccin contenant des fimbrae 987P, caractérisée par l'étape dans laquelle on transforme E. Coli avec un vecteur de clonage selon la revendication 9.

Fig. 1

Par Locus

pBTA 599

8.4 kb

Hp — Hpa I
S — Sma I
E — Eco RI
H — Hind III
P — Pst I
Pv — Pvu II

FIG 2

$_p$BTA 201

987 P  CLONE  (60.4 kb)

B    Bam HI
H    Hind III
C    Cla I
S     Sal I
x     Xho I

6kb

EP 0 242 359 B1